# EUROPEAN PATENT SPECIFICATION

(11) **EP 3 040 029 B1**
(45) Date of publication and mention of the grant of the patent: **26.12.2018**
(21) Application number: 14840621.8
(22) Date of filing: 26.08.2014
(51) Int. Cl.: A61B 8/00, A61B 6/04, A61B 6/00, A61B 8/08, B25J 3/00, B25J 13/08

(54) **MEDICAL IMAGING SYSTEM WITH MECHANICAL ARM**
MEDIZINISCHES BILDGEBUNGSSYSTEM MIT MECHANISCHEM ARM
SYSTÈME D'IMAGERIE MÉDICALE AYANT UN BRAS MÉCANIQUE

(30) Priority: 29.08.2013 CN 201320532822 U
(43) Date of publication of application: 06.07.2016
(73) Proprietor: Telefield Medical Imaging Limited, Hong Kong (CN)
(72) Inventor: ZHENG, Yongping, Hong Kong (CN); CHEUNG, James Chung Wai, Hong Kong (CN); MAK, Tak Man, Hong Kong (CN)
(74) Representative: Hellmich, Wolfgang
(86) International application number: PCT/CN2014/085196
(87) International publication number: WO 2015/027898

(56) References cited:
- EP-A1- 2 875 782
- WO-A1-2013/119610
- WO-A1-2014/015747
- CN-A- 101 610 721
- CN-A- 101 959 642
- CN-A- 102 652 675
- CN-U- 203 468 632
- JP-A- 2004 237 082
- US-A1- 2002 133 098
- US-A1- 2005 267 368
- US-A1- 2006 293 598
- US-A1- 2007 205 785
- US-A1- 2010 148 021
- US-A1- 2010 204 578
- US-A1- 2011 021 914
- US-B1- 6 425 865

## Description

### TECHNICAL FIELD

The present application relates to a medical equipment field, in particular relates to a medical imaging system with a mechanical arm.

### BACKGROUND

At present, when conducting a three-dimensional imaging on a part of the patient by a medical imaging system, such part can be diagnosed and analyzed according to the imaging results. For example, an ultrasound imaging or an X - ray imaging can be used to diagnose the scoliosis of the patient, or other parts of the patient. When conducting a three-dimensional imaging on a part of the patient by an existing medical imaging system, the operator, such as a doctor and so on, should manipulate the probe to scan such part. However, in this way, the operation of the probe is rather inconvenient and easy to aggravate the fatigue of the operator, such as a doctor and so on.

WO 2013/119610 A1 teaches a system for ultrasound scanning. This system includes an ultrasound transducer assembly configured to generate ultrasound scan data indicative of surface features of bony anatomy.

US 2011/0021914 A1 teaches a three-dimensional (3D) ultrasound imaging system for assessing scoliosis. The system comprises an ultrasound scanner to capture ultrasound images. A spatial sensor records the position and orientation of the captured ultrasound images. A software module marks features of vertebra in the captured ultrasound images.

US 2002/0133098 A1 teaches a contour mapping system useful as a spine analyser. The system includes a probe for application to a user's hand with the outer tip of at least one finger of the hand movable along the outer surface of the person's spine or other object whose contour is to be mapped.

### SUMMARY

The technical problem of the present application is to provide a medical imaging system with a more convenient probe operation.

This problem is solved by the subject matter of claim 1.

Developments are the subject matters of the dependent claims.

According to one aspect, a medical imaging system with a mechanical arm is provided, including a support, a probe, the mechanical arm, and a controller. The mechanical arm is mounted on the support. The probe is mounted at one end away from the support, of the mechanical arm. The controller can control the mechanical arm to drive the probe for conducting a multi-degree-of-freedom movement.

In the medical imaging system with a mechanical arm according to the present application, the mechanical arm includes a fixing part mounted on the support, a motion arm in transmission connection with the fixing part, and a clamping mechanism in transmission connection with one end away from the fixing part, of the motion arm. The probe is mounted on the clamping mechanism.

In the medical imaging system with a mechanical arm according to the present application, the mechanical arm further includes a first transmission mechanism, via which the motion arm is in transmission connection with the fixing part. The first transmission mechanism further includes a first motor mounted on the fixing part, a second motor mounted on an output shaft of the first motor, a third motor mounted on an output shaft of the second motor. The output shaft of the first motor and the output shaft of the second motor are arranged mutually perpendicular to each other. The motion arm is mounted on an output shaft of the third motor.

In the medical imaging system with a mechanical arm according to the present application, the mechanical arm further includes a second transmission mechanism, via which the clamping mechanism is in transmission connection with one end away from the fixing part, of the motion arm. The second transmission mechanism further includes a fourth motor mounted on one end away from the fixing part, of the motion arm; and a fifth motor mounted on an output shaft of the fourth motor. The output shaft of the fourth motor and the output shaft of the fifth motor are arranged mutually perpendicular to each other. The clamping mechanism is mounted on an output shaft of the fifth motor.

In the medical imaging system with a mechanical arm according to the present application, the motion arm includes a first motion arm in transmission connection with the fixing part, a second motion arm in transmission connection with the first motion arm, and a third motion arm in transmission connection with the second motion arm, one end of the third motion arm away from the second motion arm is in transmission connection with the clamping mechanism.

In the medical imaging system with a mechanical arm according to the present application, the mechanical arm further includes a third transmission mechanism, via which the second motion arm is in transmission connection with the first motion arm. The third transmission mechanism further includes a sixth motor mounted on the first motion arm. The second motion arm is mounted on an output shaft of the sixth motor. The second motion arm and the output shaft of the sixth motor are arranged mutually perpendicular to each other.

In the medical imaging system with a mechanical arm according to the present application, the mechanical arm further includes a fourth transmission mechanism, via which the third motion arm is in transmission connection with the second motion arm. The fourth transmission mechanism further includes a seventh motor mounted on the second motion arm. The third motion arm is mounted on an output shaft of the seventh motor. The third motion arm and the output shaft of the seventh motor are arranged mutually perpendicular to each other.

In the medical imaging system with a mechanical arm according to the present application, the medical imaging system further includes a position sensor and a pressure sensor mounted on the mechanical arm, the controller can control a movement of the mechanical arm according to signals detected by the position sensor and the pressure sensor.

In the medical imaging system with a mechanical arm according to the present application, the clamping mechanism includes a connection element in transmission connection with the motion arm, a pair of clamping bodies extending from two opposite sides of the connection element and toward each other. The probe is clamped between the pair of clamping bodies.

In the medical imaging system with a mechanical arm according to the present application, the support has a vertical structure and the mechanical arm is arranged on one side of the support.

By implementing the medical imaging system with a mechanical arm according the present application, following benefits can be obtained. The medical imaging system can control the mechanical arm to drive the probe for conducting a multi-degree-of-freedom movement, thus reducing the work strength of operating the probe by the operator, such as a doctor and so on and alleviating their fatigue. Secondly, the medical imaging system employs a mechanical arm which can improve the repeatability and accuracy of measurement. Furthermore, the medical imaging system adopts the position sensor to collect the spatial position of the probe to control the movement route of the probe in real time with supports from the information provided by the collected images. In additional, the medical imaging system adopts the pressure sensor to monitor the acting force of the mechanical arm, thus effectively avoiding application of excessive force.

### BRIEF DESCRIPTION OF THE DRAWINGS

Hereinafter, embodiments of present invention will be described in detail with reference to the accompanying drawings, wherein:
Fig.1 is a three-dimensional structure of the medical imaging system with a mechanical arm according to a first preferable embodiment of the present application.
Fig.2 is an enlarged drawing of part A in Fig.1.
Fig.3 is a structural schematic diagram of the mechanical arm when it clamps with probe.
Fig.4 is an exploded view of the mechanical arm shown in Fig.1.
Fig.5 is a three-dimensional structure of the medical imaging system with a mechanical arm according to a second preferable embodiment of the present application.

### DETAILED DESCRIPTION OF THE PREFERRED EMBODIMENT

These technical features, objects and effects of present application will be better understood from the following description and drawings.

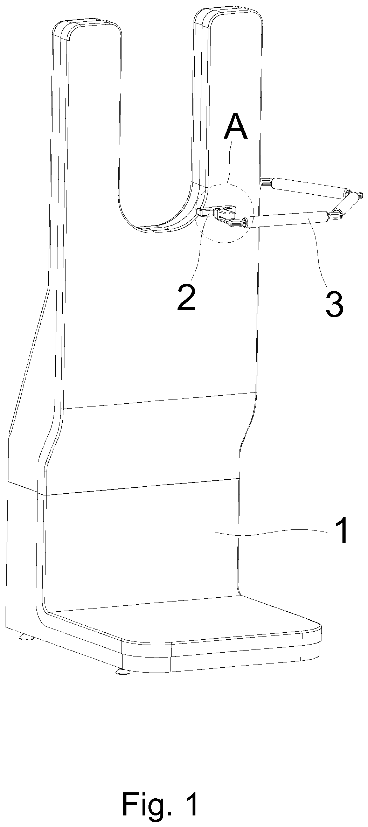

As shown in Fig.1, the medical imaging system with a mechanical arm according to a first preferable embodiment of the present application includes a support 1, a probe 2, the mechanical arm 3, a controller (unshown), a position sensor (unshown), a pressure sensor (unshown).

As shown in Fig.1, the support 1 has a vertical structure. The mechanical arm 3 is arranged on one side of the support 1. In the present embodiment, when the patient stands facing the support 1, the mechanical arm 3 is on the right side of the patient. The probe 2 is mounted on one end away from the support 1, of the mechanical arm 3. In the present embodiment, the probe 2 is an ultrasonic probe, which is used for imaging the spine of the patient. The installation height of the mechanical arm 3 is roughly matched with the spine position of the human body. In other embodiments of the present application, the probe 2 can also be used for imaging other parts of the patient.

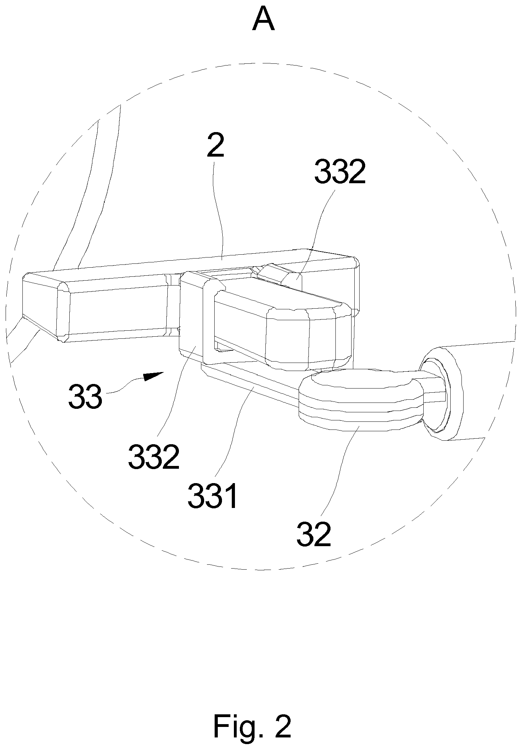

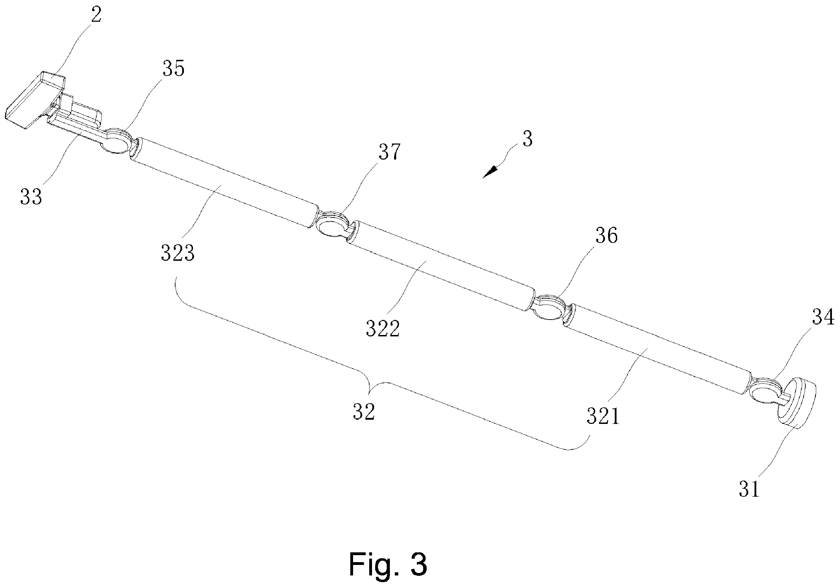

As shown in Fig.2-4, the mechanical arm 3 includes a fixing part 31, a motion arm 32, a clamping mechanism 33, a first transmission mechanism 34, a second transmission mechanism 35, a third transmission mechanism 36 and a fourth transmission mechanism 37. The fixing part 31 has a hollow cylinder structure, and is fixed on the side of the support 1. One end of the fixing part 31 far away from the support 1 is in transmission connection with the motion arm 32. The motion arm 32 includes a first motion arm 321, a second motion arm 322, and a third motion arm 323. The first motion arm 321 has a hollow rod structure, one end of which is in transmission connection with the fixing part 31, and the other end of which is in transmission connection with the second motion arm 322. The second motion arm 322 has a hollow rod structure, one end of which is in transmission connection with the first motion arm 321, and the other end of which is in transmission connection with the third motion arm 323. The third motion arm 323 has a hollow rod structure, one end of which is in transmission connection with the second motion arm 322, and the other end of which is in transmission connection with the clamping mechanism 33. The clamping mechanism 33 includes a connection element 331 and a clamping body 332. The connection element 331 has a bar structure, and is in transmission connection with the third motion arm 323. A pair of clamping bodies 332 extending from two opposite sides of the connection element 331 and toward each other is arranged. The probe 2 is clamped between the pair of clamping bodies 332.

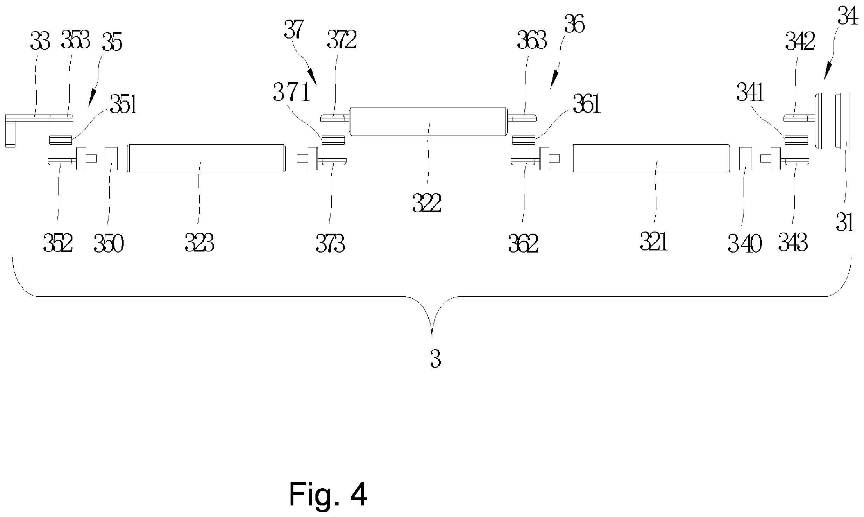

As shown in Fig.4, the first transmission mechanism 34 is used for realizing the transmission connection between the first motion arm 321 and the fixing part 31. The first transmission mechanism 34 includes a first motor (unshown), a second motor 341, a first gear set 342, a second gear set 343 and the third motor 340. The first motor is mounted inside the fixing part 31. The second motor 341 is in transmission connection with the output shaft of the first motor via the first gear set 342. The third motor 340 is in transmission connection with the output shaft (unshown) of the second motor 341 via the second gear set 343. The first motion arm 321 is mounted on the output shaft (unshown) of the third motor 340. In the present embodiment, the output shaft of the first motor and the output shaft of the second motor 341 are arranged mutually perpendicular to each other. The output shaft of the second motor 341 and the output shaft of the third motor 340 are arranged mutually perpendicular to each other.

As shown in Fig.4, the second transmission mechanism 35 is used for realizing the transmission connection between the third motion arm 323 and the connection element 331. The second transmission mechanism 35 includes a fourth motor 350, a fifth motor 351, a third gear set 352 and a fourth gear set 353. The fourth motor 350 is mounted inside one end of the third motion arm 323. The fifth motor 351 is in transmission connection with the output shaft of the fourth motor 350 via the third gear set 352. The connection element 331 is in transmission connection with the output shaft (unshown) of the fifth motor 351 via the fourth gear set 353. In the present embodiment, the output shaft of the fourth motor 350 and the output shaft of the fifth motor 351 are arranged mutually perpendicular to each other.

As shown in Fig.4, the third transmission mechanism 36 is used for realizing the transmission connection between the first motion arm 321 and the second motion arm 322. The third transmission mechanism 36 includes a sixth motor 361, a fifth gear set 362 and a sixth gear set 363. The sixth motor 361 is in transmission connection with the first motion arm 321 via the fifth gear set 362. The second motion arm 322 is in transmission connection with the output shaft (unshown) of the sixth motor 361 via the sixth gear set 363. In the present embodiment, the output shaft of the sixth motor 361 and the second motion arm 322 are arranged mutually perpendicular to each other.

As shown in Fig.4, the fourth transmission mechanism 37 is used for realizing the transmission connection between the second motion arm 322 and the third motion arm 323. The fourth transmission mechanism 37 includes a seventh motor 371, a seventh gear set 371 and an eighth gear set 373. The seventh motor 371 is in transmission connection with the second motion arm 322 via the seventh gear set 371. The third motion arm 323 is in transmission connection with the output shaft (unshown) of the seventh motor 371 via the eighth gear set 373. In the present embodiment, the output shaft of the seventh motor 371 and the third motion arm 323 are arranged mutually perpendicular to each other.

In the present embodiment, the motion arm 32 is provided with three arms. In the other embodiments of the present application, there are other optional numbers of arms in the motion arm 32. In such a way, corresponding transmission mechanisms can be added between the two adjacent arms.

The position sensor and the pressure sensor are respectively mounted on the motion arm 32. Both of the position sensor and the pressure sensor are mounted on the second motor 341. The controller can control the operations of the first transmission mechanism 34, the second transmission mechanism 35, the third transmission mechanism 36 and the fourth transmission mechanism 37, respectively according to the signals detected by the position sensor and the pressure sensor, such as to enable the mechanical arm 3 to drive the probe 2 for conducting a multi-degree-of-freedom movement, thus reducing the work strength of operating the probe by the operator, such as a doctor and so on and alleviating their fatigue. In the present embodiment, the mechanical arm 3 can drive the probe 2 for conducting a six-degree-of-freedom movement. The position sensor can collect the spatial position of the probe 2, and the controller can control the movement route of the probe in real time based on the probe spatial position together with the information provided by the collected images. The pressure sensor can monitor the acting force of the mechanical arm, thus effectively avoiding application of excessive force. In the present embodiment, the scanning route of the probe 2 on the patient's spine can also be controlled according to a predefined movement route of the mechanical arm 3.

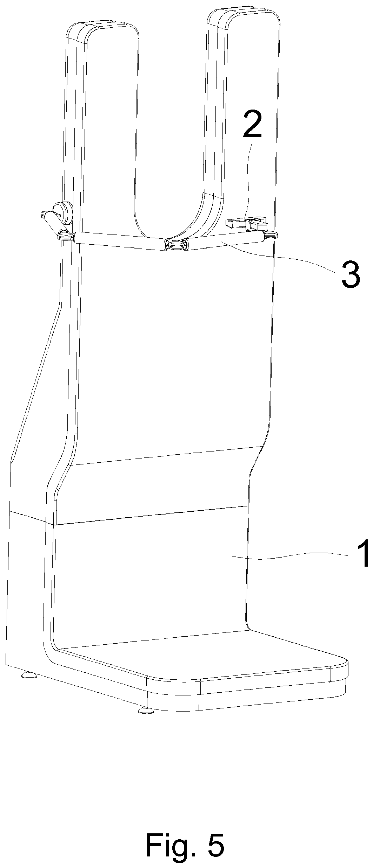

As shown in Fig.5, the second preferable embodiment of the present application has provided a medical imaging system with a mechanical arm, which is different from the first preferable embodiment in that, the mechanical arm 3 is mounted on the position of the support 1. In the present embodiment, when the patient stands facing the support 1, the mechanical arm 3 is on the left side of the patient.

## Claims

1. A medical imaging system, with a mechanical arm (3) including
a position sensor and a pressure sensor mounted on the mechanical arm (3), said pressure sensor being to monitor the acting force of the mechanical arm,
a patient support (1) adapted to be vertical in use for a patient to stand facing the patient support (1),
a probe (2), wherein the mechanical arm (3) is mounted on the patient support (1), the probe (2) is mounted at one end away from the patient support (1), of the mechanical arm (3),
a controller, wherein the controller can control the mechanical arm (3) to drive the probe (2) for implementing a multi-degree-of-freedom movement route for imaging of a spine according to signals detected by the position sensor and the pressure sensor, and wherein the position sensor is to collect the spatial position of the probe.

2. The medical imaging system with a mechanical arm (3) according to claim 1, wherein the mechanical arm (3) includes a fixing part (31) mounted on the patient support (1), a motion arm (32) in transmission connection with the fixing part (31), and a clamping mechanism (33) in transmission connection with one end away from the fixing part (31), of the motion arm (32), wherein the probe (2) is mounted on the clamping mechanism (33).

3. The medical imaging system with a mechanical arm (3) according to claim 2, wherein the mechanical arm (3) further includes a first transmission mechanism (34), via which the motion arm (32) is in transmission connection with the fixing part (31), a first transmission mechanism (34) further includes a first motor mounted on the fixing part (31), a second motor (341) mounted on an output shaft of the first motor, a third motor (340) mounted on an output shaft of the second motor (341), wherein the output shaft of the first motor and the output shaft of the second motor (341) are arranged mutually perpendicular to each other, the motion arm (32) is mounted on an output shaft of the third motor (340).

4. The medical imaging system with a mechanical arm (3) according to claim 2 or 3, wherein the mechanical arm (3) further includes a second transmission mechanism (35), via which the clamping mechanism (33) is in transmission connection with one end away from the fixing part (31), of the motion arm (32); wherein a second transmission mechanism (35) further includes a fourth motor (350) mounted on one end away from the fixing part (31), of the motion arm (32) and a fifth motor (351) mounted on an output shaft of the fourth motor (350), the output shaft of the fourth motor (350) and the output shaft of the fifth motor (351) are arranged mutually perpendicular to each other, the clamping mechanism (33) is mounted on an output shaft of the fifth motor (351).

5. The medical imaging system with a mechanical arm (3) according to one of the claims 2 to 4, wherein the motion arm (32) includes a first motion arm (321) in transmission connection with the fixing part (31), a second motion arm (322) in transmission connection with the first motion arm (321), and a third motion arm (323) in transmission connection with the second motion arm (322), one end of the third motion arm (323) away from the second motion arm (322) is in transmission connection with the clamping mechanism (33).

6. The medical imaging system with a mechanical arm (3) according to claim 5, wherein the mechanical arm (3) further includes a third transmission mechanism (36), via which the second motion arm (322) is in transmission connection with the first motion arm (321), the third transmission mechanism (36) further includes a sixth motor (361) mounted on the first motion arm (321), the second motion arm (322) is mounted on an output shaft of the sixth motor (361), the second motion arm (322) and the output shaft of the sixth motor (361) are arranged mutually perpendicular to each other.

7. The medical imaging system with a mechanical arm (3) according to claims 5 or 6, wherein the mechanical arm (3) further includes a fourth transmission mechanism (37), via which the third motion arm (323) is in transmission connection with the second motion arm (322), the fourth transmission mechanism (37) further includes a seventh motor (371) mounted on the second motion arm (322), the third motion arm (323) is mounted on an output shaft of the seventh motor (371), the third motion arm (323) and the output shaft of the seventh motor (371) are arranged mutually perpendicular to each other.

8. The medical imaging system with a mechanical arm (3) according to one of the claims 2 to 7, wherein the clamping mechanism (33) includes a connection element (331) in transmission connection with the motion arm (32), and a pair of clamping bodies (332) extending from two opposite sides of the connection element (331) and toward each other, wherein the probe (2) is clamped between the pair of clamping bodies (332).

9. The medical imaging system with a mechanical arm (3) according to of the claims 1 to 8, wherein the mechanical arm (3) is arranged on one side of the patient support (1).

## Patentansprüche

1. Medizinisches Bildgebungssystem mit einem mechanischen Arm (3), mit:
einem Positionssensor und einem Drucksensor, die auf dem mechanischen Arm (3) montiert sind, wobei der Drucksensor bestimmt ist, die wirkende Kraft des mechanischen Arms zu überwachen,
einer Patientenabstützvorrichtung (1), die für eine vertikale Verwendung an einem Patienten angepasst ist, der der Patientenabstützvorrichtung (1) zugewandt steht,
einem Messkopf (2), wobei der mechanische Arm (3) auf der Patientenabstützvorrichtung (1) montiert ist, wobei der Messkopf (2) an dem von der Patientenabstützvorrichtung (1) entfernten Ende des mechanischen Arms (3) montiert ist,
einer Steuerung, wobei die Steuerung den mechanischen Arm (3) steuern kann, um den Messkopf (2) anzutreiben, um den Messkopf (2) zum Ausführen einer Bewegungsroute mit mehreren Freiheitsgraden zum Abbilden einer Wirbelsäule gemäß von dem Positionssensor und dem Drucksensor erfassten Signalen zu steuern, wobei der Positionssensor die räumliche Position des Messkopfs (2) erfassen soll.

2. Das medizinische Bildgebungssystem mit mechanischem Arm (3) gemäß Anspruch 1, wobei der mechanische Arm (3) ein Befestigungsteil (31), das auf der Patientenabstützvorrichtung (1) montiert ist, einen Bewegungsarm (32) in Übertragungsverbindung mit dem Befestigungsteil (31) und einen Klemmmechanismus (33) in Übertragungsverbindung mit einem von dem Befestigungsteil (31) entfernten Ende des Bewegungsarms (32) umfasst, wobei der Messkopf (2) auf dem Klemmmechanismus (33) montiert ist.

3. Das medizinische Bildgebungssystem mit mechanischem Arm (3) gemäß Anspruch 2, wobei der mechanische Arm (3) ferner einen ersten Übertragungsmechanismus (34) umfasst, über den der Bewegungsarm (32) in Übertragungsverbindung mit dem Befestigungsteil (31) steht, wobei der erste Übertragungsmechanismus (34) ferner einen ersten Motor umfasst, der auf dem Befestigungsteil (31) montiert ist, einen zweiten Motor (341), der auf einer Abtriebswelle des ersten Motors montiert ist, einen dritten Motor (340), der auf einer Abtriebswelle des zweiten Motors (341) montiert ist, wobei die Abtriebswelle des ersten Motors und die Abtriebswelle des zweiten Motors (341) senkrecht zueinander angeordnet sind, wobei der Bewegungsarm (32) auf einer Abtriebswelle des dritten Motors (340) montiert ist.

4. Das medizinische Bildgebungssystem mit mechanischem Arm (3) gemäß Anspruch 2 oder 3, wobei der mechanische Arm (3) ferner einen zweiten Übertragungsmechanismus (35) umfasst, über den der Klemmmechanismus (33) in Übertragungsverbindung mit einem von dem Befestigungsteil (31) entfernten Ende des Bewegungsarms (32) steht; wobei der zweite Übertragungsmechanismus (35) ferner einen vierten Motor (350) umfasst, der auf einem von dem Befestigungsteil (31) entfernten Ende des Bewegungsarms (32) montiert ist und einen fünften Motor (351), der auf einer Abtriebswelle des vierten Motors montiert ist, wobei die Abtriebswelle des vierten Motors (350) und die Abtriebswelle des fünften Motors (351) senkrecht zueinander angeordnet sind, wobei der Klemmmechanismus (33) auf einer Abtriebswelle des fünften Motors (351) montiert ist.

5. Das medizinische Bildgebungssystem mit mechanischem Arm (3) gemäß einem der Ansprüche 2 bis 4, wobei der Bewegungsarm (32) einen ersten Bewegungsarm (321) in Übertragungsverbindung mit dem Befestigungsteil (31), einen zweiten Bewegungsarm (322) in Übertragungsverbindung mit dem ersten Bewegungsarm (321), und einen dritten Bewegungsarm (323) in Übertragungsverbindung mit dem zweiten Bewegungsarm (322) umfasst, wobei sich ein von dem zweiten Bewegungsarm (322) entferntes Ende des dritten Bewegungsarms (323) in Übertragungsverbindung mit dem Klemmmechanismus (33) befindet.

6. Das medizinische Bildgebungssystem mit mechanischem Arm (3) gemäß Anspruch 5, wobei der mechanische Arm (3) ferner einen dritten Übertragungsmechanismus (36) umfasst, über den sich der zweite Bewegungsarm (322) in Übertragungsverbindung mit dem ersten Bewegungsarm (321) befindet, wobei der dritten Übertragungsmechanismus (36) ferner einen sechsten Motor (361) umfasst, der auf dem ersten Bewegungsarm (321) montiert ist, wobei der zweite Bewegungsarm (322) auf einer Abtriebswelle des sechsten Motors (361) montiert ist, wobei der zweite Bewegungsarm (322) und die Abtriebswelle des sechsten Motors (361) senkrecht zueinander angeordnet sind.

7. Das medizinische Bildgebungssystem mit mechanischem Arm (3) gemäß Anspruch 5 oder 6, wobei der mechanische Arm (3) ferner einen vierten Übertragungsmechanismus (37) umfasst, über den sich der dritte Bewegungsarm (323) in Übertragungsverbindung mit dem zweiten Bewegungsarm (322) befindet, wobei der vierte Übertragungsmechanismus (37) ferner einen siebten Motor (371) umfasst, der auf dem zweiten Bewegungsarm (322) montiert ist, wobei der dritte Bewegungsarm (323) auf einer Abtriebswelle des siebten Motors (371) montiert ist, wobei der dritte Bewegungsarm (323) und die Abtriebswelle des siebten Motors (371) senkrecht zueinander angeordnet sind.

8. Das medizinische Bildgebungssystem mit mechanischem Arm (3) gemäß einem der Ansprüche 2 bis 7, wobei der Klemmmechanismus (33) ein Verbindungselement (331) in Übertragungsverbindung mit dem Bewegungsarm (32) und ein Paar Klemmkörper (332), die von zwei gegenüberliegenden Seiten des Verbindungselements (331) hervor- und aufeinander zuragen, umfasst, wobei der Messkopf (2) zwischen den beiden Klemmkörpern (332) eingeklemmt ist.

9. Das medizinische Bildgebungssystem mit mechanischem Arm (3) gemäß einem der vorangehenden Ansprüche, wobei der mechanische Arm (3) auf einer Seite der Patientenabstützvorrichtung (1) angeordnet. Ist.

## Revendications

1. Système d'imagerie médicale ayant un bras mécanique (3) comprenant
un capteur de position et un capteur de pression fixés sur le bras mécanique (3), ledit capteur de pression servant à surveiller la force d'actionnement du bras mécanique,
un support de patient (1) conçu pour être vertical en fonctionnement afin de permettre à un patient de se tenir debout en face du support de patient (1),
une sonde (2) dans lequel le bras mécanique (3) est fixé sur le support de patient (1), la sonde (2) est fixée au niveau d'une extrémité s'écartant du support de patient (1) du bras mécanique (3),
un système de commande, dans lequel le système de commande peut commander le bras mécanique (3) de façon à entraîner la sonde (2) en vue de réaliser une route de mouvement à multiples degrés de liberté afin de réaliser une image d'une colonne vertébrale en fonction des signaux détectés par le capteur de position et le capteur de pression et dans lequel le capteur de position sert à recueillir la position dans l'espace de la sonde.

2. Système d'imagerie médicale ayant un bras mécanique (3) selon la revendication 1, dans lequel le bras mécanique (3) comprend une partie de fixation (31) fixée sur le support de patient (1), un bras mobile (32) en connexion de transmission avec la partie de fixation (31) et un mécanisme de serrage (33) en connexion de transmission avec une extrémité s'écartant de la partie de fixation (31) du bras mobile (32), dans lequel la sonde (2) est fixée sur le mécanisme de serrage (33).

3. Système d'imagerie médicale ayant un bras mécanique (3) selon la revendication 2, dans lequel le bras mécanique (3) comprend en outre un premier mécanisme de transmission (34) via lequel le bras mobile (32) est en connexion de transmission avec la partie de fixation (31), un premier mécanisme de transmission (34) comprend en outre un premier moteur fixé sur la partie de fixation (31), un second moteur (341) fixé sur un arbre de sortie du premier moteur, un troisième moteur (340) fixé sur un arbre de sortie du second moteur (341) dans lequel l'arbre de sortie du premier moteur et l'arbre de sortie du second moteur (341) sont agencés mutuellement perpendiculairement l'un par rapport à l'autre, le bras mobile (32) étant fixé sur un arbre de sortie du troisième moteur (340).

4. Système d'imagerie médicale ayant un bras mécanique (3) selon la revendication 2 ou 3, dans lequel le bras mécanique (3) comprend en outre un second mécanisme de transmission (35) via lequel le mécanisme de serrage (33) est en connexion de transmission avec une extrémité s'écartant de la partie de fixation (31) du bras mobile (32) ; dans lequel un second mécanisme de transmission (35) comprend en outre un quatrième moteur (350) fixé sur une extrémité s'écartant de la partie de fixation (31) du bras mobile (32) et un cinquième moteur (351) fixé sur un arbre de sortie du quatrième moteur (350), l'arbre de sortie du quatrième moteur (350) et l'arbre de sortie du cinquième moteur (351) étant agencés mutuellement perpendiculairement l'un par rapport à l'autre, le mécanisme de serrage (33) étant fixé sur un arbre de sortie du cinquième moteur (351).

5. Système d'imagerie médicale ayant un bras mécanique (3) selon l'une quelconque des revendications 2 à 4, dans lequel le bras mobile (32) comprend un premier bras mobile (321) en connexion de transmission avec la partie de fixation (31), un second bras mobile (322) en connexion de transmission avec le premier bras mobile (321) et un troisième bras mobile (323) en connexion de transmission avec le second bras mobile (322), une extrémité du troisième bras mobile (323) s'écartant du second bras mobile (322) étant en connexion de transmission avec le mécanisme de serrage (33).

6. Système d'imagerie médicale ayant un bras mécanique (3) selon la revendication 5, dans lequel le bras mécanique (3) comprend en outre un troisième mécanisme de transmission (36) via lequel le second bras mobile (322) est en connexion de transmission avec le premier bras mobile (321), le troisième mécanisme de transmission (36) comprend en outre un sixième moteur (361) fixé sur le premier bras mobile (321), le second bras mobile (322) étant fixé sur un arbre de sortie du sixième moteur (361), le second bras mobile (322) et l'arbre de sortie du sixième moteur (361) étant agencés mutuellement perpendiculairement l'un par rapport à l'autre.

7. Système d'imagerie médicale ayant un bras mécanique (3) selon la revendications 5 ou 6, dans lequel le bras mécanique (3) comprend en outre un quatrième mécanisme de transmission (37) via lequel le troisième bras mobile (323) est en connexion de transmission avec le second bras mobile (322), le quatrième mécanisme de transmission (37) comprend en outre un septième moteur (371) fixé sur le second bras mobile (322), le troisième bras mobile (323) est fixé sur un arbre de sortie du septième moteur (371), le troisième bras mobile (323) et l'arbre de sortie du septième moteur (371) sont agencés mutuellement perpendiculairement l'un par rapport à l'autre.

8. Système d'imagerie médicale ayant un bras mécanique (3) selon l'une quelconque des revendications 2 à 7, dans lequel le mécanisme de serrage (33) comprend un élément de connexion (331) en connexion de transmission avec le bras mobile (32) et une paire de corps de serrage (332) s'étendant hors des deux côtés opposés de l'élément de connexion (331) et en direction l'un de l'autre, dans lequel la sonde (2) est serrée entre la paire de corps de serrage (332).

9. Système d'imagerie médicale ayant un bras mécanique (3) selon l'une quelconque des revendications 1 à 8, dans lequel le support de patient (1) a une structure verticale et le bras mécanique (3) est agencé sur un côté du support de patient (1).
